# EUROPÄISCHE PATENTSCHRIFT

(11) **EP 2 381 237 B1**
(45) Veröffentlichungstag und Bekanntmachung des Hinweises auf die Patenterteilung: **19.06.2019**
(21) Anmeldenummer: 10160828.9
(22) Anmeldetag: 23.04.2010
(51) Int. Cl.: A61M 15/00, G01N 15/02

(54) **Verfahren und Vorrichtung zur Bestimmung der Arbeitsweise einer Dosiervorrichtung**
Method and apparatus for determining the operation of a metering device
Procédé et appareil de détermination du type de travail d'un dispositif de dosage

(43) Veröffentlichungstag der Anmeldung: 26.10.2011
(60) Teilanmeldung: 19163944.2
(73) Patentinhaber: Boehringer Ingelheim microParts GmbH, 44227 Dortmund (DE)
(72) Erfinder: Golberg, Christian, 55216 Ingelheim (DE); Zumblick, Ole, 55216 Ingelheim (DE)
(74) Vertreter: Simon, Elke Anna Maria

(56) Entgegenhaltungen:
- EP-B1- 0 667 168
- WO-A1-02/059574
- WO-A2-03/000429
- WO-A2-03/055539
- US-A1- 2004 258 278
- US-A1- 2005 001 054
- US-A1- 2005 068 528
- US-A1- 2005 238 588

## Beschreibung

Die Erfindung betrifft ein Verfahren zur Bestimmung der Arbeitsweise einer Dosiervorrichtung, welche zur Erzeugung einer bestimmten Einzeldosis eines Aerosols dient und das Aerosol Partikel eines Wirkstoffs enthält, wobei die Partikel des Wirkstoffs mit einem Trägermedium zur Einzeldosis des Aerosols gemischt werden.

Die Behandlung insbesondere von chronisch-obstruktiven Lungenerkrankungen wie Asthma bronchiale oder COPD mittels inhalierten Aerosolen erlaubt eine gezielte Arzneitherapie, da der Wirkstoff mittels geeigneten Dosiervorrichtungen direkt an den pharmakologischen Zielort gebracht werden kann. Voraussetzung ist allerdings, dass die inhalierten Tröpfchen bzw. Partikel des Wirkstoffs lungengängig sind. Nur Partikel eines bestimmten Größenbereiches können an den beabsichtigten Wirkort, nämlich die tiefen Verästelungen der Lunge (Bronchiolen, Alveolen) gelangen, um dort den gewünschten therapeutischen Effekt zu entfalten.

Zu inhalierende Partikel mit einer Größe von etwa 1 bis 10 µm werden üblicherweise als lungengängig angesehen. Innerhalb dieses Bereiches gibt es wiederum Partikel, die primär durch Trägheitsimpaktion bereits im Mund- und Rachenraum abgeschieden werden und Partikel, welche hauptsächlich durch die Schwerkraft beeinflusst werden und daher für eine Abscheidung im Respirationstrakt als ideal zu bezeichnen sind. Unterschreiten die Partikel allerdings eine bestimmte Größe, so können diese wieder ausgeatmet werden und tragen zu einer gewünschten Ablagerung des Wirkstoffs in der Lunge (Lungendeposition) somit nicht bei. Zur inhalativen Applikation von Wirkstoffen werden als Dosiervorrichtungen heutzutage überwiegend treibgasgetriebene Dosieraerosole, Pulverinhalatoren sowie Zerstäuber bzw. Vernebler eingesetzt. Dabei wird bspw. bei Zerstäubern eine bestimmte Menge an (Arznei)Wirkstoff in einem geeigneten Reservoir gelagert, bei der Nutzung eine Teilmenge aus dem Wirkstoffvorrat entnommen, mittels einer Düse zu einem Aerosol zerstäubt und als Einzeldosis bereitgestellt. Die Einzeldosis wird über eine bestimmte Zeitdauer erzeugt bzw. bereitgestellt und kann dann in einem Trägermedium transportiert werden. Trägermedium ist bspw. die von einem Patienten eingeatmete Umgebungsluft.

Trotz ihrer scheinbaren Einfachheit ist die korrekte Verwendung derartiger Dosiervorrichtungen schwierig, da nach den vorliegenden Erfahrungen die meisten Patienten diese nicht korrekt bedienen. Dies rührt unter anderem daher, dass sie nicht dazu in der Lage sind, das Auslösen der Dosiervorrichtung mit dem Inhalieren zu synchronisieren und somit nicht zum richtigen Zeitpunkt inhalieren oder weil sie schlicht nicht tief genug inhalieren. Die Folge ist eine nur unzureichende Lungendeposition des Wirkstoffs. Das Problem vergrößert sich verständlicherweise bei Patienten wie Kindern, älteren Menschen und Patienten mit verringerter Respirationsfähigkeit oder verringerter manueller Fähigkeit. Die genannten Probleme können durch Dosiervorrichtungen verringert werden, deren Zeitdauer für die Erzeugung der Einzeldosis ausreichend lang bemessen ist.

Die Arbeitsweise einer Dosiervorrichtung sollte einerseits möglichst wenig vom jeweiligen Bediener (Patienten) abhängen, um immer eine gleiche, reproduzierbare Abgabe an Wirkstoff zu gewährleisten, andrerseits hängt aber sogar bei Verwendung einer Dosiervorrichtung in korrekter Weise deren Wirksamkeit (erzielbare Lungendeposition des dosierten Wirkstoffs) in großem Umfang auch von der durch die Dosiervorrichtung abgegebenen Partikelgrößenverteilung ab, welche ein Maß für die Aerosol- bzw. Wirkstoffpartikel liefert, die innerhalb eines bestimmten, lungengängigen Größenbereiches liegen sollte (wie oben bereits ausgeführt).

Die Partikelgrößenverteilung (Feinpartikelanteil) der durch eine Dosiervorrichtung erzeugten Aerosol-Einzeldosis (Sprühwolke) und die Zeitdauer für die Erzeugung einer solchen Einzeldosis (Sprühzeit, d.h. Zeit, in der die Sprühwolke aus der Dosiervorrichtung heraustritt) sind wesentliche Qualitätskriterien für eine Dosiervorrichtung.

Aus der DE 101 36 554 A1 sind ein Verfahren zur Bestimmung der Partikelgrößenverteilung in einem Aerosol und eine Vorrichtung zur Durchführung des Verfahrens bekannt. Darin wird vorgeschlagen, die Partikelgröße von Aerosolpartikeln gleichzeitig bzw. einander nachgeschaltet mittels der in der Partikelmesstechnik bekannten Laserbeugungsmethode (auch Laserdiffraktometrie genannt) und der in der Fachwelt anerkannten Kaskadenimpaktor-Methode zu messen. Dadurch soll ermöglicht werden, die Zuverlässigkeit der Ergebnisse des Laserbeugungsverfahrens auf die des Kaskadenimpaktors anzugleichen und so die Vorteile des schnellen Laserbeugungsverfahrens mit der Genauigkeit der ansonsten zeitintensiven Kaskadenimpaktor-Methode zu verbinden. Um den tatsächlich vorliegenden Bedingungen - insbesondere der hohen Luftfeuchte- im Mund-Hals-Rachenraum des Menschen gerecht zu werden, also möglichst realitätsnahe Messbedingungen zu schaffen, wird überdies vorgeschlagen, das Trägermedium mit einem Konditioniermittel vorzukonditionieren.

In der DE 10 2007 036 412 A1 wird eine Vorrichtung zur Prüfung der Dichtigkeit des Mundstücks eines Inhalators vorgeschlagen. Ferner ist aus der EP 0 667 168 B1 ein Inhalationsübungsgerät bekannt, dass einen Patienten trainieren soll, wirkungsvoll einen Aerosolinhalator über einen längeren Zeitraum zu verwenden. Unter anderem wird vorgeschlagen, Mittel zum Aufzeichnen der Betätigungszeit des Inhalators sowie der Dauer der Inhalation und des Atemanhaltens vorzusehen, um das Timing bzw. die Zeiteinstellung der Betätigung des Inhalators berechnen zu können.

US 2005/001054 A1 und WO 03/055539 A2 beziehen sich auf eine Vorrichtung zur Bestimmung der Arbeitsweise von Dosiervorrichtungen.

Der Erfindung liegt die Aufgabe zu Grunde, ein Verfahren bereitzustellen, mit dem zeit- und kostengünstig die Arbeitsweise einer Dosiervorrichtung geprüft werden kann. Des Weiteren liegt der Erfindung die Aufgabe zugrunde, eine Vorrichtung zur Durchführung des Verfahrens bereit zu stellen.

Die oben genannten Aufgaben werden mit den Merkmalen von Anspruch 1 und 6 gelöst.

Vorteilhafte Ausführungen beziehungsweise Weiterbildungen der Erfindung sind den jeweiligen Unteransprüchen entnehmbar.

Die Erfindung geht aus von einem Verfahren zur Bestimmung der Arbeitsweise einer Dosiervorrichtung, welche zur Erzeugung einer bestimmten Einzeldosis eines Aerosols dient und das Aerosol Partikel eines Wirkstoffs enthält, wobei die Partikel des Wirkstoffs mit einem Trägermedium zur Einzeldosis des Aerosols gemischt werden.

Erfindungsgemäß ist vorgesehen, dass wenigstens sowohl die in der Einzeldosis vorhandene Partikelgrößenverteilung (Feinpartikelanteil) als auch die Zeitdauer für die Erzeugung der Einzeldosis (Sprayzeit) ermittelt werden. Durch diese neuartige Verknüpfung der Messung bzw. Ermittlung von Feinpartikelanteil und Sprayzeit sind mit nur einem Messvorgang wesentliche Qualitätskriterien einer solchen Dosiervorrichtung ermittelbar, was eine vergleichsweise schnelle und kostengünstige Qualitätsprüfung in der Produktion und nicht zuletzt auch bei der Entwicklung derartiger Dosiervorrichtungen erlaubt.

Gemäß einer ersten Weiterbildung der Erfindung ist vorgesehen, dass eine Wegmessung durchgeführt wird, welche einen zur Erzeugung der Einzeldosis zurückzulegenden Verfahrweg wenigstens eines beweglichen Bauteils der Dosiervorrichtung misst. Die meisten Dosiervorrichtungen weisen wenigstens ein Bauteil auf, welches bei Erzeugung einer Einzeldosis einen bestimmten Weg durchfährt. Ein solches Bauteil kann beispielsweise der Sprühknopf einer Sprühdose sein, der zur Erzeugung einer Einzeldosis um einen bestimmten Weg heruntergedrückt werden muss oder die Patrone eines Inhalators, in der Wirkstoff bevorratet ist und welche bei Erzeugung einer bestimmten Einzeldosis ebenfalls einen bestimmten Weg durchläuft. Eine solche Wegmessung ist vergleichsweise einfach und zuverlässig durchzuführen und bringt daher einen einfachen und kostengünstigen Prüfaufbau mit sich. Zudem lassen sich über den gemessenen Verfahrweg über die Bauteilgeometrie Rückschlüsse auf das ausgebrachte Wirkstoff-Volumen ziehen. Ferner kann bei Abweichungen von einem gegebenen Verfahrweg-Sollwert darauf geschlossen werden, dass Bauteildefekte in der Dosiereinrichtung vorliegen bzw. Bauteil- und/oder Montagetoleranzen überschritten wurden.

Eine zweite Weiterbildung der Erfindung sieht vor, dass bei der Wegmessung auch eine Ermittlung der Zeitdauer für den Verfahrweg des wenigstens einen Bauteils erfolgt. Eine solche Ermittlung bzw. Berechnung der Zeitdauer kann beispielsweise über einen geeigneten Auswertungsalgorithmus erfolgen. Dies ermöglicht auf einfache Weise zusätzliche Rückschlüsse, ob Systemfehler vorliegen. Entspricht zwar ein gemessener Verfahrweg der erwarteten Norm, nicht jedoch die Verfahrweg-Zeit, so kann dies bspw. darauf hindeuten, dass die Reibung im System bspw. durch zu enge Passungen überhöht oder aufgrund von Leckage zu niedrig ist.

Gemäß einer weiteren Ausgestaltung des Erfindungsgedankens erfolgt die Messung der Partikelgrößenverteilung mittels der Laserbeugung (Laserdiffraktometrie). Erfindungsgemäß erfolgt die Weg-/Zeitmessung mittels der Laser-Triangulation. Diese sind bekannte und zuverlässige Messverfahren. Deren Einsatz trägt daher zu gesicherten Messergebnissen bei. Diese Verfahren brauchen ansonsten an dieser Stelle nicht näher erläutert zu werden.

Zur Nachbildung eines möglichst realitätsnahen Messaufbaus wird das Trägermedium vorkonditioniert. Bevorzugt wird hierbei Luft als Trägermedium und als Konditioniermittel Wasser verwendet.

Der Nachbildung eines möglichst realitätsnahen Messaufbaus ist es auch zuträglich, wenn zumindest das Trägermedium einer Absaugung unterzogen und die Absaugung mit dem Messvorgang synchronisiert wird.

Die Erfindung betrifft aber auch eine Vorrichtung zur Durchführung des Verfahrens. Diese umfasst erfindungsgemäß wenigstens eine Messzelle zur Aufnahme des Trägermediums und der Einzeldosis des Aerosols, wenigstens eine erste Messeinrichtung zur Messung der Partikelgrößenverteilung in der Einzeldosis und wenigstens eine zweite Messeinrichtung, deren aufgenommene Messwerte eine Ermittlung der Zeitdauer für die Erzeugung der Einzeldosis (Sprayzeit) zulassen.

Die zweite Messeinrichtung misst den Verfahrweg wenigstens eines Bauteils der Dosiervorrichtung, welcher zur Erzeugung der Einzeldosis zurückgelegt werden muss.

Zweckmäßig wird bei der Wegmessung durch die zweite Messeinrichtung auch die Zeitdauer für den Verfahrweg des wenigstens einen Bauteils ermittelt. Genauer gesagt misst ein Sensor der zweiten Messeinrichtung den Verfahrweg und ein der zweiten Messeinrichtung zugeordneter Auswertealgorithmus errechnet bzw. ermittelt die Zeit. Es erfolgt also quasi eine Weg-/Zeitmessung.

Dabei können vorteilhaft als erste Messeinrichtung ein auf den Grundlagen der Laserbeugung arbeitender Partikelgrößenanalysator und als zweite Messeinrichtung ein Triangulations-Laser eingesetzt werden. Kostengünstig und bewährt haben sich hier als Mess-Laser Helium-Neon-Laser, die bspw. rotes Laserlicht mit einer Wellenlänge von 632,8 nm und hoher Kohärenzlänge aussenden können.

Die verwendeten Laser sind dabei vorzugsweise so zu positionieren, dass die durch die Laser erzeugten Laserstrahlen in etwa in einem Winkel von 90° zueinander stehen. Dies ermöglicht einen unkomplizierten Prüfaufbau. Je nach zu prüfender Dosiervorrichtung kann es jedoch zweckmäßig sein, die Laser nicht nur so anzuordnen, dass deren erzeugte Laserstrahlen in etwa senkrecht zueinander stehen, sondern es kann auch vorteilhaft sein, wenn die Laser so zueinander positioniert sind, dass sich deren erzeugte Laserstrahlen in einer Ebene kreuzen.

Zur Nachbildung einer möglichst realitätsnahen Prüfumgebung kann wenigstens eine Konditioniereinrichtung vorgesehen sein, die mit der Messzelle in fluidischer Verbindung steht und in der Messzelle vorkonditioniertes Trägermedium bereitstellt. Bevorzugt wird als Trägermedium Luft und als Konditioniermittel Wasser verwendet. Dabei kann zusätzlich eine Messung der Luftfeuchte bspw. durch einen Feuchtesensor vorgesehen sein, wobei nach Art eines Regelkreises eine gewünschte Luftfeuchte eingestellt werden kann.

Entsprechend trägt auch zu einem realitätsnahen Prüfaufbau bei, wenn wenigstens eine Absaugeinrichtung vorgesehen ist, welche mit der Messzelle in fluidische Verbindung bringbar ist, derart, dass während eines Messvorgangs das in der Messzelle befindliche, aus Trägermedium und Wirkstoffpartikel bestehende Fluid, zumindest jedoch das Trägermedium abgesaugt wird. Dabei kann die fluidische Verbindung zeitlich synchronisiert mit dem Messvorgang erfolgen. Hierzu kann beispielsweise ein ansteuerbares Magnetventil eingesetzt werden.

Weitere Vorteile und Ausgestaltungen der Erfindung werden anhand von einem bevorzugten Ausführungsbeispiel deutlich, was mit Hilfe der beiliegenden Figuren näher erläutert werden soll. Dabei bedeuten
- Fig. 1: ein äußerst schematische Darstellung der erfindungsgemäßen Vorrichtung zur Durchführung des erfindungsgemäßen Verfahrens,
- Fig. 2: ein Weg-Zeit-Diagramm, welches bei einem Messvorgang mittels der Vorrichtung erhalten werden kann,
- Fig. 3: eine mit der Vorrichtung erstellte, normierte Messwertreihe aus einer Fähigkeitsuntersuchung (Typ1-Studie), welche den jeweils gemessenen Feinpartikelanteil zeigt und
- Fig. 4: eine mit der Vorrichtung erstellte, normierte Messwertreihe, welche auch die bei der Fähigkeitsuntersuchung gemäß Fig.3 jeweils ermittelte Sprühzeit zeigt.

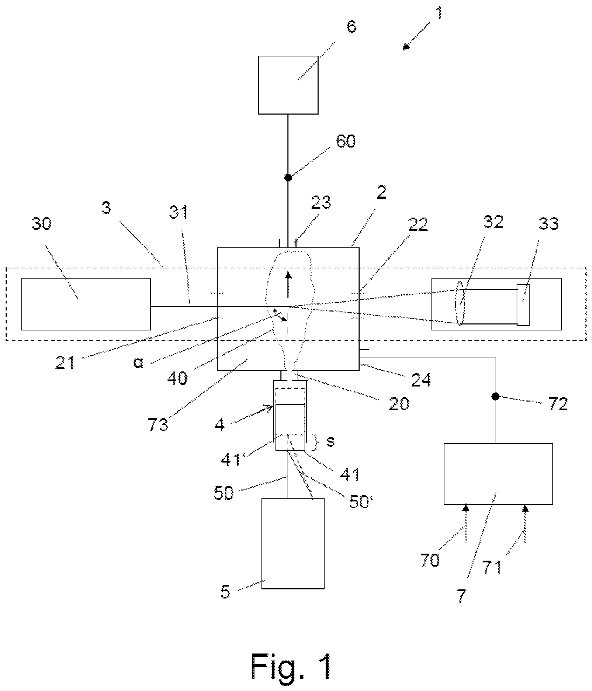

In Fig. 1 ist ein bevorzugtes Ausführungsbeispiel einer Vorrichtung 1 zur Durchführung des erfindungsgemäßen Verfahrens prinziphaft dargestellt. Die Vorrichtung 1 umfasst eine Messzelle 2, die gegenüber dem Umfeld im Wesentlichen licht- und luftdicht abgeschlossen ist. Die Messzelle dient zur Aufnahme einer Sprühwolke 40 eines Wirkstoffs (Einzeldosis), welche durch eine Dosiervorrichtung 4 erzeugt wurde. Die Dosiervorrichtung 4 ist dazu mit einem Einlass 20 der Messzelle 2 luftdicht verbunden. Die Messzelle 2 weist ferner zwei lichtdurchlässige Fenster 21 und 22 auf, durch die ein von einem Partikelgrößen-Messgerät 3 erzeugter Laserstrahl 31 hindurchtreten kann. Genauer gesagt wird der Laserstrahl 31 durch einen He-Ne-Laser 30 erzeugt. Das vom Laser 30 ausgesendete Licht fällt durch das Fenster 21 ins Innere der Messzelle 2 und trifft dort auf die erzeugte Sprühwolke 40. Dabei wird das einfallende Licht an den Partikeln der Sprühwolke 40, welche für das Licht ein Hindernis darstellen, gebeugt. Das Streulicht, welches durch die Beugung des einfallenden Laserlichtes an den Partikeln der Sprühwolke 40 generiert wird, verlässt die Messzelle 2 durch das Fenster 22, wird dann durch eine Sammellinse 32 gebündelt und einem Halbleiter-Detektor 33 zur Auswertung durch geeignete Auswerteverfahren, wie bspw. das Mie-Verfahren oder die Fraunhofer Methode, zugeführt. Da die Partikelgrößenmessung mittels der Laserbeugung an sich bekannt ist, wird nicht näher darauf eingegangen. Auch bedarf es keiner näheren Erläuterung, dass entsprechende Auswerte-Rechnersysteme natürlich vorzusehen sind, die in der Fig.1 jedoch nicht näher dargestellt sind.

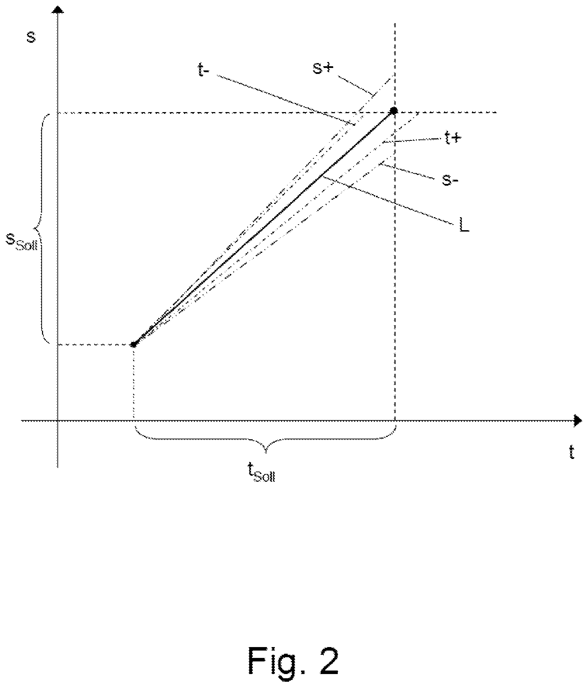

Bei dem erfindungsgemäßen Verfahren wird als Dosiervorrichtung 4 vorzugsweise ein Inhalator der Marke Respimat® gemessen. Dies ist ein treibgasfreier Inhalator mit Erzeugung einer sanften und lang anhaltenden Sprühwolke 40. Offenbart ist ein solcher Inhalator beispielsweise in der WO 97/12687. Eine genauere Erläuterung seines Aufbaus erfolgt daher nicht. Die Dosiervorrichtung 4 weist dabei als bewegliches Bauteil 41 eine Aluminium-Patrone auf, in dem ein Wirkstoff als Suspension enthalten ist und welches als Wirkstoff-Reservoir dient. Bei jeder Erzeugung einer Spraywolke 40 (Einzeldosis) vollführt das Bauteil 41 einen durch die Bauteilgeometrie der Dosiervorrichtung 4 vorbestimmten Verfahrweg s, der in der Fig. 1 durch die Ausgangsposition des Bauteils 41 und seine Endposition (41') begrenzt ist. Hinter dem Boden des Bauteils 41 ist wiederum ein Messgerät 5 in Form eines Triangulations-Lasers angeordnet. Das Messgerät 5 dient quasi zu einer Weg-/Zeitmessung und sendet einen Laserstrahl 50 aus, der am Boden des Bauteils 41 diffus reflektiert wird (50') und somit eine an sich bekannte Laser-Triangulationsmessung ermöglicht. Hierdurch wird sowohl der Verfahrweg s des Bauteils 41 gemessen als auch über einen dem Messgerät 5 zugeordneten Auswertealgorithmus eine dafür benötigte Zeit t errechnet bzw. ermittelt (vgl. Fig.2). Zur Erzeugung des Laserstrahls 50 dient wiederum vorzugsweise ein He-Ne-Laser. Bevorzugt sind die Messzelle 2, die daran angeschlossene Dosiereinrichtung 4 sowie die Messgeräte 3 und 5 in einer horizontalen Ebene angeordnet.

Aus der Fig.1 ist ferner eine Absaugeinrichtung 6 ersichtlich, welche permanent eingeschaltet sein kann und fluidisch mit einem Auslass 23 der Messzelle 2 verbunden ist. Wird nun durch Betätigung der Dosiereinrichtung 4 (eine entsprechende Vorrichtung zur Erleichterung der Betätigung im Messaufbau in Form eines externen Schalters ist nicht dargestellt) in der Messzelle 2 eine Sprühwolke 40 erzeugt, so wird ein Messvorgang ausgelöst und nur für die Dauer des Messvorgangs durch ein angedeutetes, elektrisch ansteuerbares Magnetventil eine fluidische Verbindung zwischen Absaugeinrichtung 6 und Messzelle 2 hergestellt. Dies führt dazu, dass eine Strömung der Sprühwolke 40 in Richtung der Absaugeinrichtung 6 erzeugt wird (s. Pfeil), welche in etwa quer zum Laserstrahl 31 des Partikelgrößen-Messgerätes 3 verläuft. Durch die Strömung wird ein Einatmen der Sprühwolke 40 durch einen Patienten simuliert.

Ferner ist eine Konditioniereinrichtung 7 gezeigt, welche eine Luftzufuhr 70 und eine Wasserzufuhr 71 aufweist, wobei Luft- und Wasser zu einer befeuchteten Luft mit einstellbarem Sättigungsgrad bei vorgebbarer Temperatur vermischt werden. Die Konditioniereinrichtung 7 ist mit einem Einlass 24 der Messzelle 2 verbunden, so dass befeuchtete Luft 73 als vorkonditioniertes Trägermedium in der Messzelle 2 vorgehalten werden kann. Hierdurch soll der feuchte Mund-Rachenraum eines Patienten möglichst realitätsnah nachgebildet werden. Zur Überwachung der Einstellungen kann ein angedeuteter Feuchtesensor 72 vorgesehen sein.

Schließlich sei noch erwähnt, dass die Messgeräte 3 und 5 vorzugsweise so angeordnet sind, dass deren Laserstrahlen 31 bzw. 50 etwa in einem rechten Winkel α zueinander stehen, was zu einem einfacheren Messaufbau und genaueren Messergebnissen führt. Im vorliegenden Ausführungsbeispiel erfolgt die Anordnung sogar so, dass die Laserstrahlen 31 und 50 in etwa in einer Ebene liegen und sich deren gedachte Verlängerungen kreuzen.

Nunmehr wird auf die Fig. 2 Bezug genommen. Dort ist prinziphaft ein Weg-/Zeit-Diagramm ersichtlich, welches aus der Messung des Messgerätes 5 herrühren kann. Darin ist der vom Bauteil 41 zurückgelegte Verfahrweg s über der dafür benötigten Zeit t aufgetragen. Die Linie L stellt dabei den Soll-Verlauf dar, wenn also das Bauteil 41 idealerweise einen vorgeschriebenen Verfahrweg s in einer vorgeschriebenen Zeit t zurücklegt. Gibt es bei der zu prüfenden Dosiereinrichtung 4 irgendwelche Qualitätsfehler, so weicht die gemessene Linie von der Soll-Linie Soll ab. Es könnten bspw. folgende Fälle auftreten:
- Der Verfahrweg s des Bauteils 41 ist aufgrund zu hoher Fertigungs- und/oder Montagetoleranzen zu kurz (s-) oder zu lang (s+)
- Die Zeit t für den Verfahrweg s ist aufgrund sonstiger Systemfehler (bspw. defekte Düse oder sonstige Leckage) zu kurz (t-) oder zu lang (t+)

Aufgrund der vorliegenden Bauteilgeometrie kann von dem Verfahrweg s auch auf das aus der Dosiervorrichtung 4 ausgegebene Wirkstoffvolumen geschlossen werden. Gleichermaßen können von der Zeit t für den Verfahrweg s Rückschlüsse auf die Sprühzeit der Dosiervorrichtung 4 gezogen werden. Die Zeit t für den Verfahrweg s entspricht nämlich in etwa auch der Sprühzeit.

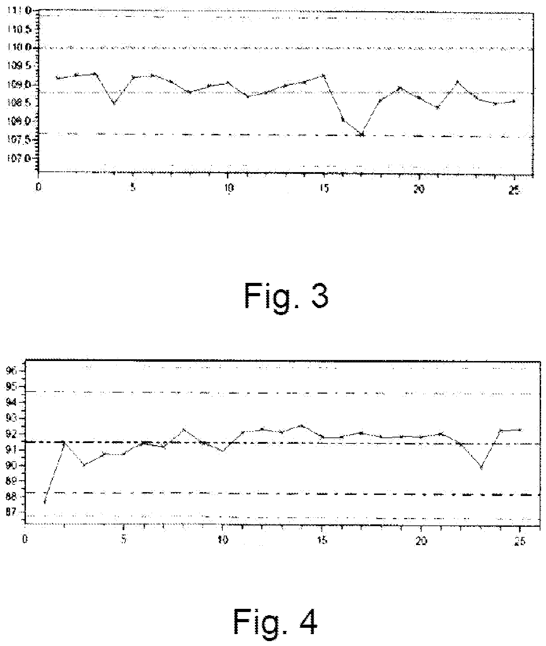

Schließlich sei auf die Fig. 3 und 4 Bezug genommen. Diese stellen den Teil der Ergebnisse einer sogenannten Fähigkeitsstudie (Typ 1) dar und zeigen, wie gut das erfindungsgemäße Verfahren Aufschluss über wesentliche Qalitätseigenschaften von Dosiervorrichtungen, beispielhaft der Dosiervorrichtung 4 geben kann bzw. wie gut die Komponenten der Messvorrichtung zusammenarbeiten.

So sind in Fig. 3 und 4 Messwerte einer Messreihe mit der erfindungsgemäßen Vorrichtung von über 25 Messungen dargestellt, wobei in Fig. 3 der Feinpartikel-Anteil qualitativ in Prozent (normiert) über der Anzahl der durchgeführten Messungen dargestellt ist. 100 Prozent bedeuten hierbei eine Erreichung der gewünschten bzw. geforderten Mindestwertes. Die erzielten Werte liegen erkennbar in einem Bereich deutlich darüber.
In Fig. 4 ist hingegen die Sprühzeit qualitativ in Prozent über der Anzahl der durchgeführten Messungen dargestellt. Hier bedeuten 100 Prozent eine idealerweise angestrebte Sprühzeit. Die erzielten Werte kommen mit einem Wert von ca. 91,5 % ziemlich nahe an diesen Zielwert heran.

Ein weiterer Teil der Fähigkeitsstudie (Typ 2), der jedoch nicht näher dargestellt ist, zeigte, dass das erfindungsgemäße Verfahren bzw. die Messvorrichtung im Wesentlichen unabhängig von einem Bedienereinfluss ist.

Es sei abschließend noch einmal darauf hingewiesen, dass das erfindungsgemäße Verfahren nicht auf die Messung von Inhalatoren beschränkt ist, sondern sich für jegliche Dosiervorrichtungen eignet, die wegbezogen arbeiten, bei denen also durch den zurückgelegten Weg eines beweglichen Bauteils eine Wirkstoff-Dosis ausgegeben wird.

### Bezugszeichenliste

- 1: Messvorrichtung
- 2: Messzelle
- 20: Einlass
- 21: Lichtfenster
- 22: Lichtfenster
- 23: Auslass
- 24: Einlass
- 3: Partikelgrößen-Messgerät
- 30: Laser des Partikelgrößen-Messgerätes
- 31: erzeugter Laserstrahl
- 32: Linse
- 33: Halbleiter-Detektor
- 4: Dosiervorrichtung
- 40: erzeugte Sprühwolke des Wirkstoffs (Einzeldosis)
- 41,41': bewegliches Bauteil der Dosiervorrichtung
- 5: Weg/Zeit-Messgerät
- 50: erzeugter Laserstrahl
- 6: Absaugeinrichtung
- 60: Magnetventil
- 7: Konditioniereinrichtung
- 70: Luftzufuhr
- 71: Wasserzufuhr
- 72: Feuchtesensor
- 73: befeuchtete Luft
- α: Winkel, in denen die Laserstrahlen der Messgeräte zueinander stehen
- s: Verfahrweg des beweglichen Bauteils
- ssoll: Soll-Verfahrweg des beweglichen Bauteils
- L: Soll-Linie im Weg-/Zeit-Diagramm
- s+: Weg-/Zeit Diagramm mit überhöhtem Weg
- s-: Weg-/Zeit Diagramm mit zu geringem Weg
- t: benötigte Zeit des beweglichen Bauteils für Zurücklegung des Verfahrwegs
- tsoll: Sollzeit des beweglichen Bauteils für Zurücklegung des Verfahrwegs
- t+: Weg-/Zeit Diagramm mit überhöhtem Zeitbedarf
- t+: Weg-/Zeit Diagramm mit zu geringem Zeitbedarf

## Patentansprüche

1. Verfahren zur Bestimmung der Arbeitsweise einer Dosiervorrichtung (4), welche (4) zur Erzeugung einer bestimmten Einzeldosis (40) eines Aerosols dient und das Aerosol Partikel eines Wirkstoffs enthält, wobei die Partikel des Wirkstoffs mit einem Trägermedium (73) zur Einzeldosis (40) des Aerosols gemischt werden, wobei wenigstens die in der Einzeldosis (40) vorhandene Partikelgrößenverteilung und eine Zeitdauer für die Erzeugung der Einzeldosis (40) ermittelt werden, wobei eine Laser-Triangulationsmessung durchgeführt wird, bei welcher ein zur Erzeugung der Einzeldosis (40) zurückzulegender Verfahrweg (s) wenigstens eines beweglichen Bauteils (41,41') der Dosiervorrichtung (4) gemessen wird und die Zeitdauer (t) für den Verfahrweg (s) ermittelt wird.

2. Verfahren nach Anspruch 1, **dadurch gekennzeichnet, dass** der Verfahrweg (s) und die Zeit (t) mit einer Soll-Verlauf-Linie (L) verglichen werden, um Qualitätsfehler der Dosiervorrichtung (4) festzustellen.

3. Verfahren nach wenigstens einem der vorhergehenden Ansprüche, **dadurch gekennzeichnet, dass** die Messung der Partikelgrößenverteilung mittels Laserbeugung erfolgt.

4. Verfahren nach wenigstens einem der vorhergehenden Ansprüche, **dadurch gekennzeichnet, dass** das Trägermedium (73) befeuchtet wird.

5. Verfahren nach wenigstens einem der vorhergehenden Ansprüche, **dadurch gekennzeichnet, dass** zumindest das Trägermedium (73) einer Absaugung unterzogen und die Absaugung mit dem Messvorgang synchronisiert wird.

6. Vorrichtung (1) zur Durchführung des Verfahrens nach wenigstens einem der vorhergehenden Ansprüche, umfassend wenigstens eine Messzelle (2) zur Aufnahme des Trägermediums (73) und der Einzeldosis (40) des Aerosols, wenigstens eine erste Messeinrichtung (3) zur Messung der Partikelgrößenverteilung in der Einzeldosis (40) und wenigstens eine zweite Messeinrichtung (5), die einen Verfahrweg (s) wenigstens eines beweglichen Bauteils (41) einer Dosiervorrichtung (4) misst, welcher Verfahrweg (s) zur Erzeugung der Einzeldosis (40) zurückgelegt werden muss, und deren Messwerte eine Ermittlung der Zeitdauer (t) für die Erzeugung der Einzeldosis (40) zulassen, wobei in der zweiten Messeinrichtung ein Triangulations-Laser eingesetzt ist.

7. Vorrichtung nach Anspruch 6, **dadurch gekennzeichnet, dass** die zweite Messeinrichtung (5) über einen zugeordneten Auswertealgorithmus die Zeitdauer (t) für den Verfahrweg (s) des wenigstens einen Bauteils (41) ermittelt.

8. Vorrichtung nach wenigstens einem der Ansprüche 6 bis 7, **dadurch gekennzeichnet, dass** die erste Messeinrichtung (3) ein auf den Grundlagen der Laserbeugung arbeitender Partikelgrößenanalysator ist.

9. Vorrichtung nach Anspruch 8, **dadurch gekennzeichnet, dass** durch die Messeinrichtungen (3,5) erzeugte Laserstrahlen (31,50) in etwa in einem Winkel von 90° zueinander stehen.

10. Vorrichtung nach wenigstens einem der Ansprüche 6 bis 9, **dadurch gekennzeichnet, dass** wenigstens eine Konditioniereinrichtung (7) vorgesehen ist, die mit der Messzelle (2) in fluidischer Verbindung steht und in der Messzelle (2) vorkonditioniertes Trägermedium (73) bereitstellt.

11. Vorrichtung nach wenigstens einem der Ansprüche 6 bis 10, **dadurch gekennzeichnet, dass** eine Absaugeinrichtung (6) vorgesehen ist, welche mit der Messzelle (2) in fluidische Verbindung bringbar ist, derart, dass während eines Messvorgangs das in der Messzelle (2) befindliche Fluid abgesaugt wird.

12. Vorrichtung nach dem Anspruch 9, **dadurch gekennzeichnet, dass** die Laserstrahlen (31,50) sich in einer Ebene kreuzen.

## Claims

1. A method for determining the operation of a metering device (4), which device (4) is used to produce a specific single dose (40) of an aerosol and the aerosol contains particles of an active ingredient, wherein the particles of the active ingredient are mixed with a carrier medium (73) to form the single dose (40) of the aerosol, wherein at least the particle size distribution in the single dose (40) and a duration for producing the single dose (40) are ascertained, wherein a laser triangulation measurement is carried out, during which a travel path (s) of at least one movable component (41, 41') of the metering device (4) is measured, wherein the travel path (s) is to be followed in order to produce the single dose (40), and the duration (t) for the travel path (s) is ascertained.

2. The method according to claim 1, **characterised in that** the travel path (s) and the time (t) are compared with a desired course line (L) in order to detect quality errors of the metering device (4).

3. The method according to at least one of the preceding claims, **characterised in that** the particle size distribution is measured by means of laser diffraction.

4. The method according to at least one of the preceding claims, **characterised in that** the carrier medium (73) is humidified.

5. The method according to at least one of the preceding claims, **characterised in that** at least the carrier medium (73) is subject to suction and the suction is synchronised with the measuring process.

6. A device (1) for carrying out the method according to at least one of the preceding claims, comprising at least one measuring cell (2) for receiving the carrier medium (73) and the single dose (40) of the aerosol, at least one first measuring apparatus (3) for measuring the particle size distribution in the single dose (40), and at least one second measuring apparatus (5) which measures a travel path (s) of at least one movable component (41) of a metering device (4), which travel path (s) has to be followed in order to produce the single dose (40), and the measured values of which second apparatus make it possible to ascertain the duration (t) for producing the single dose (40), wherein a triangulation laser is used in the second measuring apparatus.

7. The device according to claim 6, **characterised in that** the second measuring apparatus (5) ascertains the duration (t) for the travel path (s) of the at least one component (41) by means of an associated evaluation algorithm.

8. The device according to at least one of claims 6 to 7, **characterised in that** the first measuring apparatus (3) is a particle size analyser which operates on the principles of laser diffraction.

9. The device according to claim 8, **characterised in that** laser beams (31, 50) generated by the measuring apparatuses (3, 5) are at an angle of approximately 90° from one another.

10. The device according to at least one of claims 6 to 9, **characterised in that** at least one conditioning apparatus (7) is provided which is in fluid communication with the measuring cell (2) and provides preconditioned carrier medium (73) in the measuring cell (2).

11. The device according to at least one of claims 6 to 10, **characterised in that** a suction apparatus (6) is provided which can be brought into fluid communication with the measuring cell (2) such that the fluid in the measuring cell (2) is sucked up during a measuring process.

12. The device according to claim 9, **characterised in that** the laser beams (31, 50) intersect in a plane.

## Revendications

1. Procédé de détermination du fonctionnement d'un dispositif de dosage (4), lequel (4) sert à la production d'une dose individuelle (40) déterminée d'un aérosol et l'aérosol contient des particules d'un principe actif, dans lequel les particules du principe actif sont mélangées avec un milieu de support (73) dans la dose individuelle (40) de l'aérosol, dans lequel au moins la distribution de la taille des particules présente dans la dose individuelle (40) et une durée de production de la dose individuelle (40) sont déterminées, dans lequel une mesure par triangulation laser est réalisée, lors de laquelle un trajet de déplacement (s), parcouru pour la production de la dose individuelle (40), d'au moins un composant mobile (41, 41') du dispositif de dosage (4) est mesuré et la durée (t) pour le trajet de déplacement (s) est déterminée.

2. Procédé selon la revendication 1, **caractérisé en ce que** le trajet de déplacement (s) et le temps (t) sont comparés avec une ligne d'évolution de consigne (L) pour constater des défauts de qualité du dispositif de dosage (4).

3. Procédé selon au moins l'une quelconque des revendications précédentes, **caractérisé en ce que** la mesure de la distribution de la taille des particules est effectuée par diffraction laser.

4. Procédé selon au moins l'une quelconque des revendications précédentes, **caractérisé en ce que** le milieu de support (73) est humidifié.

5. Procédé selon au moins l'une quelconque des revendications précédentes, **caractérisé en ce qu'**au moins le milieu de support (73) est soumis à une aspiration et l'aspiration est synchronisée avec le procédé de mesure.

6. Dispositif (1) de réalisation du procédé selon au moins l'une quelconque des revendications précédentes, comprenant au moins une cellule de mesure (2) pour la réception du milieu de support (73) et de la dose individuelle (40) de l'aérosol, au moins un premier dispositif de mesure (3) pour la mesure de la distribution de la taille des particules dans la dose individuelle (40) et au moins un deuxième dispositif de mesure (5), qui mesure un trajet de déplacement (s) d'au moins un composant mobile (41) d'un dispositif de dosage (4), lequel trajet de déplacement (s) doit être parcouru pour la production de la dose individuelle (40), et dont les valeurs de mesure permettent une détermination de la durée (t) de production de la dose individuelle (40), dans lequel un laser de triangulation est utilisé dans le deuxième dispositif de mesure.

7. Dispositif selon la revendication 6, **caractérisé en ce que** le deuxième dispositif de mesure (5) détermine la durée (t) du trajet de déplacement (s) de l'au moins un composant (41) par le biais d'un algorithme d'évaluation associé.

8. Dispositif selon au moins l'une quelconque des revendications 6 à 7, **caractérisé en ce que** le premier dispositif de mesure (3) est un analyseur de la taille des particules fonctionnant sur les bases de la diffraction laser.

9. Dispositif selon la revendication 8, **caractérisé en ce que** les rayons laser (31, 50) générés par les dispositifs de mesure (3, 5) forment à peu près un angle de 90° l'un par rapport à l'autre.

10. Dispositif selon au moins l'une quelconque des revendications 6 à 9, **caractérisé en ce qu'**au moins un dispositif de conditionnement (7) est prévu, qui est en liaison fluidique avec la cellule de mesure (2) et met à disposition un milieu de support (73) préconditionné dans la cellule de mesure (2).

11. Dispositif selon au moins l'une quelconque des revendications 6 à 10, **caractérisé en ce qu'**un dispositif d'aspiration (6) est prévu, lequel peut être mis en liaison fluidique avec la cellule de mesure (2) de sorte que pendant un procédé de mesure, le fluide se trouvant dans la cellule de mesure (2) est aspiré.

12. Dispositif selon la revendication 9, **caractérisé en ce que** les rayons laser (31, 50) se croisent dans un plan.
